**Europäisches Patentamt**

**European Patent Office**

**Office européen des brevets**

(19)

(11) Numéro de publication: **0 267 861**
**A2**

(12) **DEMANDE DE BREVET EUROPEEN**

(21) Numéro de dépôt: **87440014.6**

(22) Date de dépôt: **11.03.87**

(51) Int. Cl.³: **A 41 B 13/02**

(30) Priorité: **15.10.86 FR 8614600**

(43) Date de publication de la demande:
**18.05.88 Bulletin 88/20**

(84) Etats contractants désignés:
**AT BE CH DE ES GB GR IT LI LU NL SE**

(71) Demandeur: **CELATOSE Société anonyme dite:**
**47, Rue de Bradford**
**F-59200 Tourcoing (Nord)(FR)**

(72) Inventeur: **Rollé, Alain**
**47 rue de Bradford**
**F-59202 Tourcoing (Nord)(FR)**

(74) Mandataire: **Lepage, Jean-Pierre**
**Cabinet Lepage & Aubertin Innovations et Prestations**
**23/25, rue Nicolas Leblanc B.P. 1069**
**F-59011 Lille Cédex 1 (Nord)(FR)**

(54) **Installation de fabrication de produits plats en forme.**

(57) L'invention est relative à une installation de fabrication de produits plats en forme (2) tels que notamment des changes jetables pour bébés ou pour incontinents.

L'installation (1) est constituée de différents postes de travail successifs (11 à 29) disposés en ligne dans lesquels on forme, à partir de matières diverses de base (3 à 8) en nappes bobinées, une bande continue composite adaptée aux produits à obtenir (2).

De plus, selon l'invention, elle comporte des moyens pour contrôler le défilement des nappes de matières de base (3 à 8), des moyens pour contrôler de manière synchrone la bande composite (40) ainsi formée en défilement, selon des paramètres de formes et de qualité du produit à obtenir (2), des moyens pour repérer, selon le pas "p", les produits obtenus (2) non conformes aux dits paramètres, et des moyens pour éjecter après leur découpage les produits obtenus (2) repérés non conformes.

FIG.1

-1-

L'invention est relative à une installation de fabrication de produits plats en forme tels que notamment des changes jetables pour bébés ou pour incontinents.

Elle trouvera notamment son application d'une part pour la réalisation et la construction d'une telle installation et d'autre part dans le domaine de fabrication en chaine de produits plats en forme notamment mais non exclusivement des changes jetables pour bébés ou pour incontinenets.

En effet, dans de nombreux domaines de fabrication industrielle, on cherche à optimaliser la production et à diminuer les coûts de revient des produits fabriqués. C'est notamment le cas dans la fabrication d'articles dits jetables ou à usage unique. Ce dernier critère impose obligatoirement la vente des produits fabriqués à un prix très étudié pour le consommateur qui ainsi le préférera par commodité à des produits de fonction identique plus coûteux à l'achat mais réutilisables.

Depuis ces dernières années, le développement technologique a permis de mécaniser et d'automatiser de nombreuses installations de fabrication de produits. Ces améliorations technologiques ont permis de mettre des produits sur le marché qui existent de longues dates en version réutilisable qui répondent plus aux besoins du consommateur moderne grâce à leur jetabilité.

C'est notamment le cas dans le domaine de la protection hygiénique pour bébés et pour incontinents. En effet, on utilise pour ce type de protection des couches absorbantes qui, dans un premier temps, ont été réalisées en matière textile traditionnelle. Puis, sont apparues des couches de formes simples, notamment rectangulaires, conçues en fibres cellulosiques, maintenues sur l'individu par une pièce textile en matière plastique.

La commodité d'utilisation de tels types de produits jetables et leur succès auprès du consommateur ont poussé les industriels à sophistiquer leur fabrication. C'est pourquoi, on a vu apparaitre de tels produits plats composites en forme, généralement commercialisés sous l'appellation "couches culottes ou changes jetables".

Ces nouveaux produits présentent l'avantage de regrouper toutes les fonctions en un seul produit, à savoir : confort pour l'individu, absorption efficace, étanchéité et maintien de l'humidité. C'est pourquoi, ils sont encore plus appréciés des consommateurs et des utilisateurs pour leur commodité. Toutefois, de tels perfectionnements augmentent sensiblement le coût de revient des produits ainsi fabriqués.

Afin de minimiser le coût de revient, on a donc réalisé des installations de fabrication de tels produits plats en forme, constituées de différents postes de travail successifs disposés en ligne pour un travail en continu et qui, à partir des diverses matières de base alimentant la dite installation, permettent d'obtenir en fin de chaine le produit plat composite en forme complètement façonné.

En effet, il est connu des installations de fabrication de produits plats en forme, tels que notamment des changes jetables pour bébés ou pour incontinents, constituées de différents postes de travail successifs aptes à former, à partir des matières de base en nappes bobinées par exemple, une bande continue composite présentant au moins deux couches externes, à savoir une couche continue étanche au liquide et une couche continue présentant un toucher confortable vis-à-vis de la peau, et présentant un élément, en forme de tampon, perméable et absorbant, placé entre les dites couches externes à espaces réguliers.

Ensuite, les deux couches externes et le tampon sont solidarisés entre eux pour donner de la cohérence à la bande et les produits plats en forme sont délivrés en fin d'installation par découpage de la bande continue entre chaque élément tampon.

De telles installations peuvent fonctionner selon des cadences importantes de 200 à 300 produits par minute mais il s'agit d'une production quantitative et la qualité du produit fini dépend strictement de la fiabilité des moyens mis en oeuvre dans les installations connues. Or, comme la qualité est primordiale aux yeux du consommateur et de l'utilisateur, il est courant en fin de chaine de disposer de postes de contrôle manuel afin d'éliminer avant l'empaquetage les produits fabriqués qui ne répondent pas aux critères de qualité ou qui présentent des vices de forme.

En effet, quelle que soit l'attention portée à la réalisation de telles installations de fabrication tant sur leur principe de fonctionnement que sur leurs moyens, certains paramètres de fonctionnement de l'installation feront que certains produits présenteront en fin de chaine des défauts ou anomalies. Ces derniers auront des origines diverses et on réalisera par exemple des produits finis non conformes dans les cas suivants.

A la mise en service de l'installation ou plus exactement lors de chaque redémarrage de la chaine, la vitesse de défilement de la bande continue passe d'une production zéro à un seuil de production compris entre 60 et 100 produits/mn.

Lors du fonctionnement de l'installation, les matières premières de base se présentent généralement en bobines, et lors des changements de bobines, il y a recouvrement des nappes de matière, ce qui provoquera des surépaisseurs au niveau du produit fabriqué; Ceci peut par exemple se produire sur la couche externe étanche ou encore sur la couche externe de confort.

D'autres défauts de qualité ou de forme peuvent apparaitre aussi au cours de la fabrication par exemple décalage de la bande continue en défilement vis-à-vis des différents postes, ce qui provoquera des décalages latéraux au niveau du produit fini, décalage de la coupe transversale finale, ce qui provoquera un mauvais positionnement de l'élément tampon par rapport aux côtés latéraux du produit, mauvaise mise en forme de l'élément absorbant.

De plus, dans le cas spécifique des changes jetables, il est courant d'apposer des élastiques de part et d'autre de l'élément tampon pour obtenir des fronces et pour que le produit fini épouse les formes de l'individu en le garantissant contre les fuites. Ces élastiques se présentent généralement sous la forme d'une bande méplat collée sur la couche étanche externe. C'est pourquoi, au cours de la fabrication, il se peut que cet élastique se retourne ou encore qu'il soit mal collé. Ces deux inconvénients sont d'autres défauts de qualité.

Enfin, toujours dans le cas spécifique des changes jetables, il est courant d'apposer des rubans adhésifs de chaque côté latéral à une position bien précise pour autoriser le maintien du change sur l'individu. Ces adhésifs sont un critère de qualité primordial car un change où les adhésifs sont manquants ou mal positionnés sera finalement non adapté à l'usage.

Or, de par les systèmes de pose d'adhésifs connus actuellement, il arrive assez couramment que ceux-ci soient mal positionnés ou même non apposés.

Par ailleurs, pour abaisser actuellement le coût de revient, il est important, d'une part, de réduire les causes de défaut et, d'autre part, d'augmenter encore sensiblement la cadence de production.

Si on peut minimiser les causes de défaut en réalisant une installation selon des techniques plus fiables, le contrôle manuel des produits en fin de chaine, par contre, limite la cadence ou dans le cas de cadences plus élevées, nécessite une main d'oeuvre plus importante.

Le but de la présente invention est de proposer une installation de fabrication de produits plats en forme, tels que notamment des changes jetables pour bébés ou pour incontinents, qui puisse remédier aux problèmes posés ci-dessus en proposant un système de contrôle automatique des différentes phases de fabrication avec en fin de chaine l'éjection des produits non conformes, avant leur empaquetage.

Un autre but de la présente invention est de proposer une installation de fabrication de produits plats en forme, tels que notamment des changes jetables pour bébés ou pour incontinents, qui permette de rendre plus fiable certaines opérations de fabrication, notamment la pose des adhésifs.

Un des buts de la présente invention est de proposer une installation de fabrication de produits plats en forme qui permette de contrôler le produit durant toute sa fabrication, selon des paramètres de forme et de qualité, pour obtenir finalement des produits de toute première qualité ne présentant pas de surépaisseurs de matériaux au niveau des couches externes ni de défauts, au niveau de l'élément tampon absorbant, de forme ou de positionnement, mais par contre présentant une configuration géométrique contrôlée, une bonne cohérence, des adhésifs et des élastiques bien positionnés.

D'autres buts de la présente invention apparaitront au cours de la description qui va suivre, qui n'est cependant donnée qu'à titre indicatif et qui n'a pas pour but de la limiter.

Selon la présente invention, l'installation de fabrication de produits plats en forme, tels que notamment des changes jetables pour bébés ou pour incontinents, constituée de différents postes de travail successifs en ligne et comprenant au moins :

- des moyens pour former, à partir de diverses matières de base en nappes bobinées, une bande continue composite présentant au moins deux couches externes, à savoir : une couche continue étanche au liquide, et une couche continue présentant un toucher confortable vis-à-vis de la peau, et présentant un élément en forme, perméable et absorbant placé entre les deux dites couches externes, selon un pas prédéterminé "p",

- des moyens pour solidariser au moins partiellement les deux couches externes et le dit élément absorbant entre eux, est caractérisée par le fait qu'elle comporte :

- des moyens pour contrôler le défilement des nappes de matières de base,

- des moyens pour contrôler, de manière synchrone, la bande composite ainsi formée en défilement, disposés aux différents postes de travail de l'installation, selon des paramètres de forme et de qualité du produit à obtenir,

- des moyens pour repérer, selon le pas "p", les produits obtenus non conformes aux dits paramètres,

- des moyens pour découper la bande continue ainsi formée, selon le pas "p", pour produire les produits plats en forme,

- des moyens pour éjecter, après leur découpage, les produits repérés non conformes.

Selon différentes caractéristiques de la présente invention, on éliminera de la chaine pour non conformes :

- les produits sans adhésif et/ou avec adhésif mal positionnés,

- les produits formés pendant les régimes transitoires de fonctionnement de la dite installation,

- les produits présentant une surépaisseur dans les couches externes,

- les produits dans lesquels l'élastique est retourné ou non collé,

- les produits présentant une surépaisseur et/ou une absence locale de matière au niveau de l'élément interne absorbant,

- les produits présentant une dissymétrie dans le sens transversal,

- les produits présentant un élément absorbant mal positionné par rapport aux extrémités longitudinales du produit.

L'invention sera mieux comprise à la lecture de la description suivante accompagnée des dessins en annexe qui en font partie intégrante.

La figure 1 montre schématiquement une installation de fabrication de produits plats en forme illustrant la succession des différents postes de travail en ligne.

La figure 2 montre un produit plat en forme, tel qu'un change jetable pour bébés ou pour incontinents, obtenu par une installation du type de la figure 1.

La figure 3 montre le change jetable représenté à la figure 2 sous sa forme pliée pour l'empaquetage.

Les figures 4a à 4e montrent différentes étapes successives de la fabrication du change jetable représenté à la figure 2.

Les figures 5a à 5c illustrent les différentes étapes de pose des adhésifs réalisés par l'installation de la présente invention.

La figure 6 montre schématiquement la bande continue en vue de dessus et explicite le contrôle de présence et de positionnement des adhésifs selon la présente invention.

La figure 7 montre un détail de l'installation de la présente invention qui explicite le fonctionnement de l'installation lors du changement de bobines de matières premières.

La figure 8 montre en détail la fin de la ligne de fabrication illustrant notamment la coupe, l'éjection des produits non conformes, et l'évacuation des produits finis.

L'invention vise une installation de fabrication de produits plats composites en forme. Elle sera particulièrement adaptée à la fabrication de changes jetables pour bébés ou pour incontinents mais plus généralement pourra s'appliquer à la fabrication de produits d'hygiène ou similaires.

Comme il a été rappelé dans la partie introductive de la présente demande, il est de plus en plus courant d'utiliser de tels produits qui sont conçus à usage unique et donc jetables. Ceci par exemple dans le cas des changes ou couches culottes pour bébés ou pour incontinents permet une utilisation commode en supprimant les entretiens et lavages fastidieux tout en présentant un prix de revient raisonnable.

Néanmoins, pour abaisser le coût de revient de tels produits, il est nécessaire de les fabriquer d'une façon fiable et de les produire le plus automatiquement possible tout en évitant au maximum les rebuts.

De plus, pour éviter des contrôles manuels en fin de chaine qui se répercutent sensiblement sur le prix de revient, il est intéressant de disposer d'une installation dont les contrôles se font automatiquement en provoquant le rejet en fin de chaine des produits repérés non conformes.

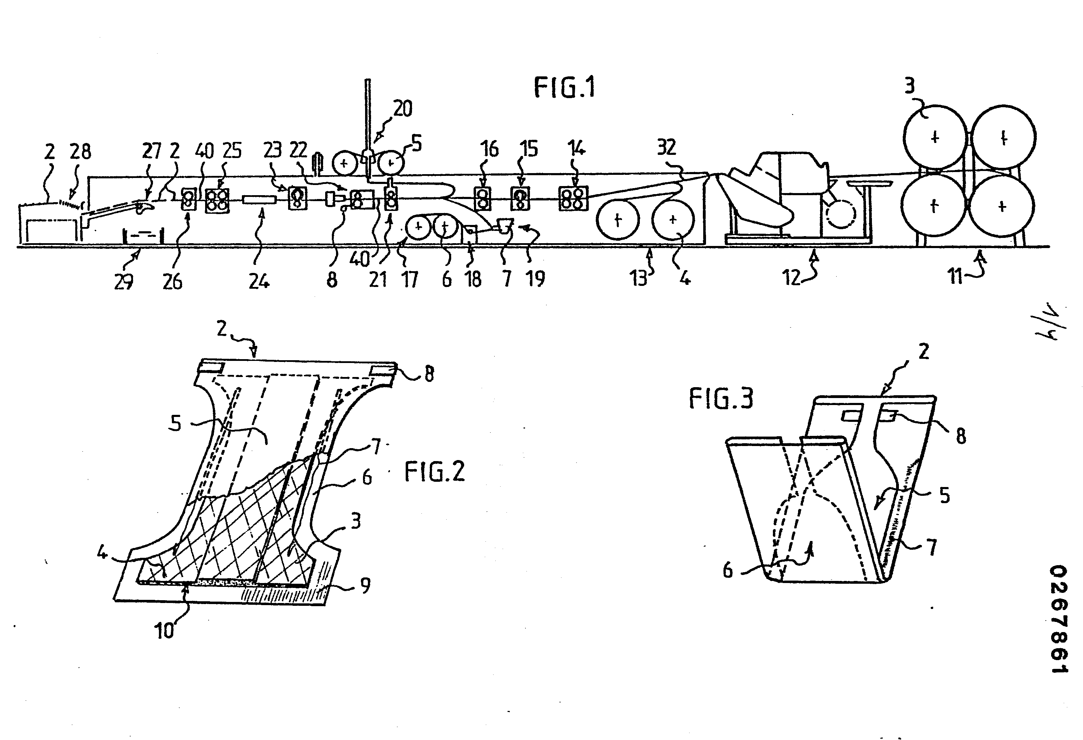

La figure 1 montre schématiquement une installation 1 de fabrication de produits plats 2 tels que notamment des changes jetables pour bébés ou pour incontinents. Cette installation 1 permet de réaliser à partir de matières diverses de base les dits produits 2 par traitement en ligne dans différents postes de travail successifs.

Pour mieux comprendre la suite de la description, nous allons préalablement décrire la constitution d'un exemple non limitatif de produits plats en forme : un change jetable tel que représenté à la figure 2. Ce croquis n'illustre qu'un type de produit, la forme du produit final et les caractéristiques pouvant naturellement être adaptées selon les besoins par l'Homme de l'Art.

Le change jetable 2 est essentiellement constitué d'un élément 10 en forme, perméable et absorbant, placé entre une première couche externe étanche au liquide 6 et une deuxième couche 5 qui elle est perméable et présente un toucher confortable vis-à-vis de la peau de l'individu.

Plus précisément, l'élément absorbant 10 est réalisé à partir de fibres cellulosiques 3 et entouré d'un voile de fibres cellulosiques 4 qui assure la cohésion de l'élément 10. En ce qui concerne les couches externes, la couche 5 est notamment réalisée en matière non tissée telle que par exemple à base de polypropylène qui présente avantageusement un toucher confortable. Par ailleurs, la couche étanche 6 est avantageusement constituée par un film de polyéthylène d'épaisseur telle qu'elle soit résistante mécaniquement et à l'allongement.

En outre, le change jetable 2 présente des élastiques 7 disposés de part et d'autre de l'élément absorbant 10 sur ses côtés latéraux afin de créer des fronces au niveau de l'entrejambe de l'individu. Selon des modes de réalisation, l'élastique pourra être soit du type caoutchouc, soit en polyuréthane et sera avantageusement

-8-

collé sur le film de polyéthylène étanche 6.

Enfin, au niveau d'un des côtés transversaux du change 2 est prévue de part et d'autre une longueur de ruban adhésif 8 repliée sur elle-même. Les caractéristiques de cet adhésif seront déterminées par l'Homme de l'Art pour qu'il soit par exemple du type enlevable tout en présentant un bon pouvoir de solidarisation des éléments.

La figure 3 montre le change jetable 2 tel qu'il vient d'être décrit plié une première fois en C, puis une seconde fois en deux afin d'occuper un minimum de place et de favoriser son rangement et emballage.

Un tel type de change peut être réalisé dans une installation 1 telle que représentée à la figure 1 qui à cet égard comporte successivement les différents postes suivants :

a) un poste d'alimentation de pâte à papier 11 conçu pour recevoir notamment trois ou quatre bobines de pâte à papier 3 qui constitueront la matière de base du tampon absorbant,

b) un poste de défibrage de la pâte 12, alimenté à partir des bobines de pâte 3, équipé d'un dispositif du type broyeur à marteau par exemple.

Après le défibrage de la pâte, on forme la nappe absorbante et à cet effet, on fait appel à des dispositifs scarificateurs conçus pour produire une nappe dont l'épaisseur est avantageusement plus grande au centre qu'aux extrémités afin d'améliorer l'absorption pour une masse donnée de fibres.

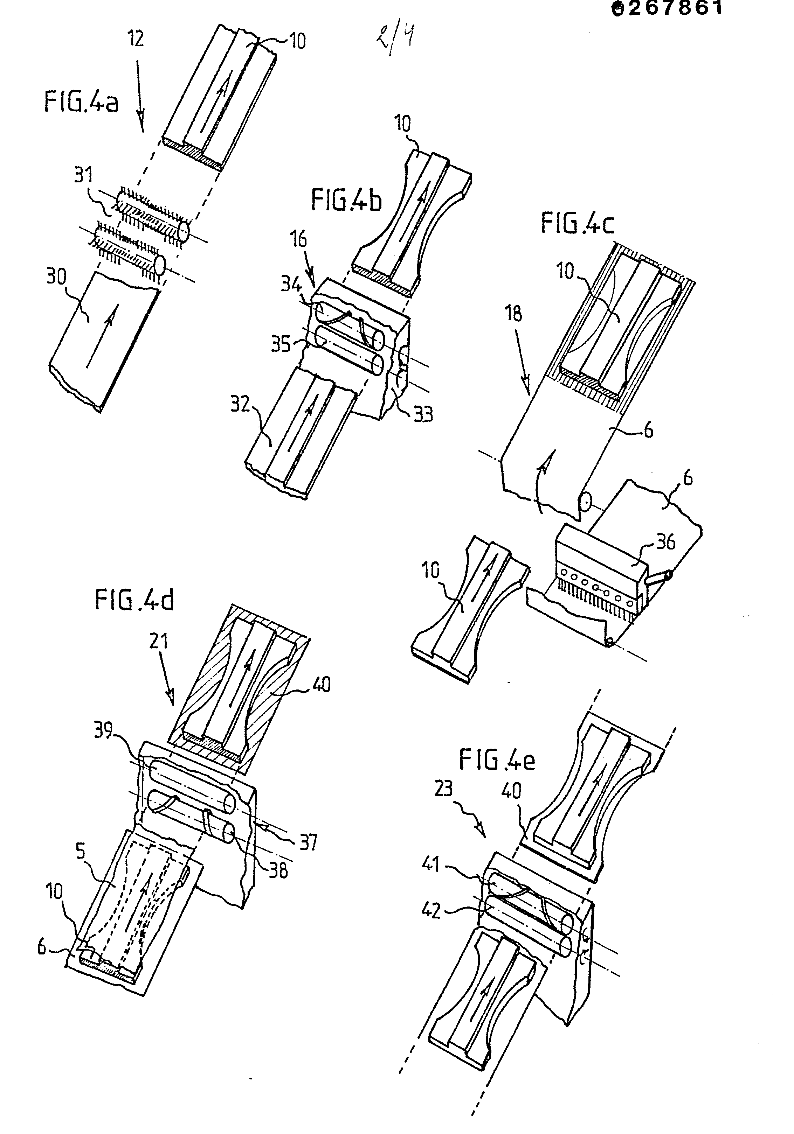

La figure 4a montre schématiquement la forme de la nappe absorbante qui formera l'élément absorbant 10. On y voit notamment la nappe de fibres 30 qui avance vers les dispositifs scarificateurs 31 adaptés aux formes du tampon ce qui donne en sortie une bande formée de nappes absorbantes.

c) un poste dévidoir 13 de voile de fibres cellulosiques bobiné 4 destiné à consolider la nappe absorbante délivrée en sortie du poste 12.

Plus précisément, à ce niveau, la nappe de matières absorbantes est déposée sur le voile cellulosique 4 qui est par exemple véhiculé sur un plan incliné et subit ensuite un pliage longitudinal en trois de sorte que le voile entoure la matière fibreuse pour donner de la cohérence à l'élément absorbant 10.

d) un poste de gaufrage 14 vers lequel est dirigée ensuite la bande en défilement formée par la nappe de fibres enveloppée du dit voile afin d'imprimer à cette dernière un gaufrage en losange qui permettra avantageusement une absorption optimisée du liquide par l'élément tampon 10.

e) un poste de découpe 15 anatomique de l'élément absorbant, tel que notamment illustré à la figure 4b.

Ainsi, la nappe 32 de fibres entourée du voile de consolidation est dirigée vers un ensemble de découpes 33 constitué au moins d'un cylindre couteau 34 et d'un contre-cylindre enclume 35.

Il est à remarquer que le couteau rotatif est dessiné spécifiquement selon les dimensions du produit et plus précisément, le développé de ce couteau correspond à la longueur du tampon à fabriquer.

f) un poste de coupe élément absorbant 16 comprenant notamment une lame droite coupant à la volée, à chaque taille, on définit le dit élément absorbant 10 unitaire.

g) un poste d'alimentation 17 de la matière de base 6 constituant substantiellement la couche externe étanche qui comporte notamment essentiellement deux bobines dévidoirs sur lesquelles sont enroulés notamment un film de polyéthylène tel que défini préalablement.

h) un poste d'encollage 18, notamment schématisé en détail à la figure 4c, qui permettra d'enduire le film étanche 6 afin de fixer postérieurement l'élément absorbant 10 sur ce film.

Selon un mode de réalisation, on dispose d'une buse 36 d'encollage multi-traits qui applique de fins traits de colle longitudinalement et essentiellement parallèles sur une des faces du film polyéthylène 6.

i) un poste de dépose des élastiques 19 dans lequel un élastique 7 est amené de chaque côté de l'élément absorbant 10.

L'élastique est ensuite solidarisé sur le film polyéthylène, soit par enduction séparée, soit en utilisant l'enduction multi-traits décrite ci-dessus.

j) un poste d'alimentation 20 en matière de base constituant substantiellement la couche externe 5 au toucher confortable, qui comporte notamment deux bobines dévidoirs permettant de diriger la nappe de non-tissé 5 vers la bande en défilement continu jusqu'alors formée par le film polyéthylène 6 sur lequel sont disposés périodiquement selon un pas "p" les éléments absorbants 10.

k) un poste de scellage des extrémités 21 dont le but est de former la bande sandwich composite 40 continue qui servira de base à la formation postérieure des changes jetables 2.

En effet, comme décrit ci-dessus, les éléments absorbants 10 unitaires sont déposés sur le film étanche 6 de polyéthylène qui arrive par le dessous des éléments 10. La nappe 5 de non-tissé est dirigée au-dessus de l'autre face des éléments tampons 10 afin que ces derniers soient pris en sandwich entre les deux couches externes formées respectivement par le film 6 et la nappe non tissée 5.

Il est à remarquer que le film étanche 6 et la nappe de non-tissé 5 ont des vitesses linéaires voisines, toutefois supérieures à celles d'arrivée des éléments tampons 10 afin de créer le pas "p" entre chaque élément tampon 10.

Dans ce poste 21 est prévue une presse anatomique 37 constituée par un cylindre presse 38 au profil adapté à l'élément tampon et un cylindre enclume 39 lisse dont les vitesses de rotation sont synchronisées par rapport à la vitesse d'avancement de la bande composite.

Ainsi, la presse anatomique 37 comprime chaque intervalle entre l'élément tampon 10 et les bords afin d'assurer le scellage de la nappe de non-tissé 5 sur film étanche de polyéthylène 6 grâce à l'encollage multi-traits précédemment déposé. La figure 4d illustre particulièrement cette opération et les hâchures obliques entourant l'élément tampon illustre les parties compressées et scellées entre elles.

A ce niveau, on a formé, grâce aux différents moyens qui viennent d'être décrits, une bande continue composite 40 présentant au moins deux couches externes, à savoir la couche 6 continue étanche au liquide et la couche 5 continue présentant un toucher confortable vis-à-vis de la peau, et présentant un élément 10 en forme, perméable et absorbant, placé entre les deux dites couches externes 5 et 6 selon un pas "p" prédéterminé. De plus, les couches externes 5 et 6 et le dit élément absorbant 10 sont solidaires au moins partiellement entre eux afin d'assurer une bonne cohérence de la bande en défilement 40.

l) un poste applicateur 22 d'adhésifs dans lesquels deux

longueurs "l" de rubans adhésifs 8 sont placées de chaque côté d'une extrémité du change par des moyens de découpe, de positionnement, et de pose des adhésifs, synchronisés avec le défilement de la bande en continu 40 et commandés selon le dit pas "p". Ces différents moyens seront décrits plus en détail dans la suite de la description.

Les adhésifs apposés servent à positionner et à maintenir le change 2 sur l'individu. Ils seront notamment constitués par une bande composite d'adhésifs et de protecteurs siliconés.

m) un poste de découpe anatomique 23 de la nappe de non-tissé 5 et du film étanche de polyéthylène 6 au niveau de l'entrejambe du change, tel que notamment illustré à la figure 4c.

Ce poste est constitué d'une manière similaire à celui utilisé pour la découpe de l'élément absorbant 10. Il comporte notamment un cylindre couteau 41 présentant des arêtes de coupe adaptées au profil à découper et un cylindre enclume 42 synchronisé sur la vitesse de défilement de la bande 40.

n) un poste de pliage en C 24 et un poste de compresseur 25 permettant de mettre en forme la bande continue en défilement 40.

o) un poste de découpe finale 26 vers lequel la bande 40 est dirigée et dans lequel cette dernière est découpée pour séparer le change jetable 2 ainsi formé de la bande continue 40.

p) un poste d'évacuation 28 des dits produits plats en forme 2 ainsi réalisés.

Par exemple le change 2 est maintenu entre deux convoyeurs pour être introduit finalement dans une chaine à palette de laquelle seront ensuite repris les produits pour leur emballage par exemple.

Jusqu'à présent, les différents postes qui ont été décrits sont généralement connus de l'Homme de l'Art et utilisent des techniques traditionnelles. Toutefois, selon l'état de la technique, les produits sont contrôlés manuellement au niveau de leur sortie au poste 26.

Selon la présente invention, pour supprimer les contrôles de qualité manuels, l'installation comporte en outre :

a) des moyens pour contrôler le défilement des nappes de matières de base 3 à 8,

b) des moyens pour contrôler, de manière synchrone, la bande composite 40 ainsi formée en défilement, disposés aux différents postes de travail de l'installation ; contrôle effectué selon des paramètres de forme et de qualité du produit à obtenir 2,

c) des moyens pour repérer, selon le pas "p", les produits obtenus non conformes aux dits paramètres,

d) des moyens pour éjecter après leur découpage les produits obtenus 2 repérés non conformes.

Les différents moyens de contrôle vont être explicités ci-dessous et seront notamment prévus pour détecter les produits non conformes selon l'un ou plusieurs des critères suivants :

- produits sans adhésif et/ou avec adhésifs mal positionnés,

- produits formés pendant les régimes transitoires du fonctionnement de l'installation,

- produits présentant une surépaisseur ou une double épaisseur dans les couches externes,

- produits dans lesquels l'élastique est mal ou pas collé,

- produits présentant une surépaisseur et/ou une absence locale de matières,

- produits présentant une dissymétrie dans le sens transversal du dit produit,

- produits présentant un élément absorbant mal centré par rapport aux extrémités longidutinales du dit produit.

Ainsi, la présente installation 1 présente des moyens 45 pour contrôler la présence des adhésifs 8 après leur pose ainsi que leur positionnement.

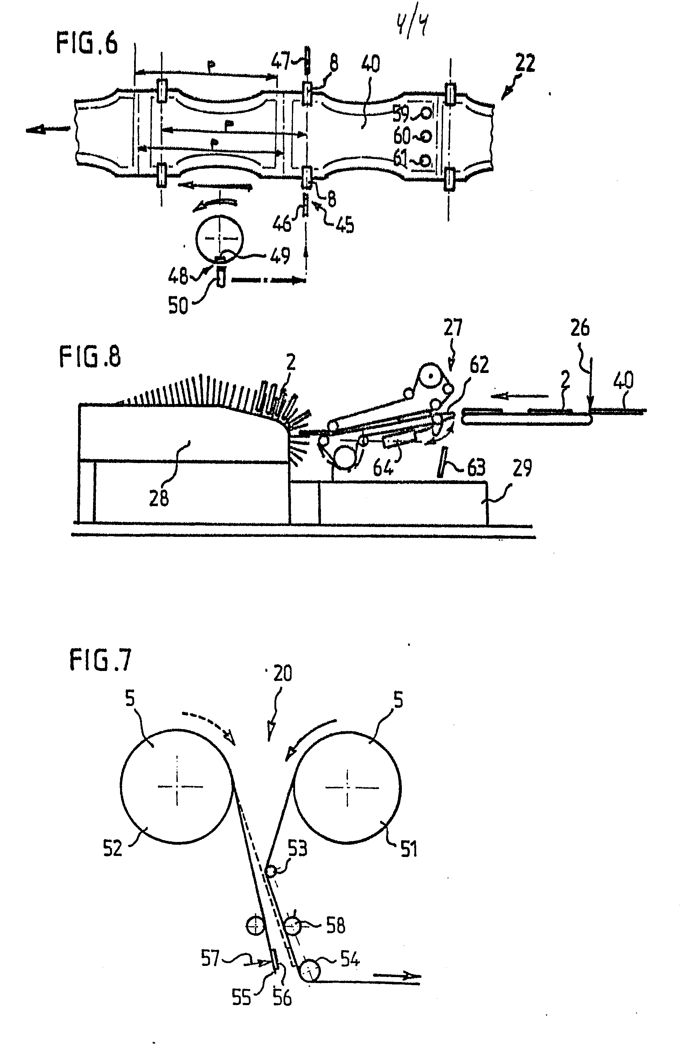

Ces moyens 45 sont notamment prévus au niveau du poste 22 de découpe d'adhésifs et leur fonctionnement est explicité au vue de la figure 6.

Plus précisément, après la pose des adhésifs, on prévoit un ensemble de cellules photo-électriques 46, 47, placées de part et d'autre de la bande en défilement 40, aptes à être occultées par la présence des adhésifs 8. Ces systèmes photo-électriques seront avantageusement constitués par des fibres optiques et des détecteurs optiques fonctionnant en barrage ou à réflexion, techniques connues de l'Homme de l'Art.

Toutefois, ces moyens 45, et plus particulièrement les cellules 46 et 47, agiront de manière synchrone par rapport au défilement de la bande 40 afin de détecter non seulement la présence de l'adhésif mais leur bon positionnement. Pour ce, on utilisera un dispositif de synchronisation 48 qui sera asservi au pas "p" des produits.

Dans un mode de réalisation avantageux, ces moyens 48 seront constitués par exemple par une roue en rotation dont le développement correspond au pas "p", portant sur sa périphérie un repère 49 qui sera détecté, par exemple, par un capteur de proximité 50, qui délivrera un top de synchronisation de lecture pour les cellules 46 et 47. Ainsi, on détectera les produits non conformes sans adhésif et/ou avec adhésifs mal positionnés avec une précision de plus ou moins 10 mm.

De plus, le produit détecté non conforme encore solidaire de part et d'autre au produit précédent et au produit suivant dans la bande 40 sera mémorisé par un circuit de traitement électronique non représenté sur les figures, qui permettra de délivrer un top d'évacuation du produit non conforme après sa phase de découpage au poste 26.

Une telle technique de repérage est traditionnelle et connue de l'Homme de l'Art du domaine considéré.

Ainsi, à chaque top de synchronisation délivré par le dispositif 48, on contrôlera selon le pas "p" la présence et le positionnement successif des adhésifs et on mémorisera les éventuels défauts.

Une autre cause d'éjection des produits non conformes résulte des régimes transitoires du fonctionnement de la dite installation. En effet, pour mettre en marche une installation pouvant délivrer jusque 400 produits/minute, il est nécessaire d'effectuer les différents réglages des postes successifs et d'accélérer progressivement l'installation.

Toutefois, pour détecter et repérer les produits non conformes formés pendant les régimes transitoires, la dite installation de fabrication 1 comporte des moyens pour contrôler la vitesse de défilement de la bande continue 40 afin d'évacuer et d'éjecter systématiquement les produits non conformes fabriqués pendant ces dits régimes transitoires.

Par ailleurs, les matières de base 3 à 8 se présentent généralement en nappes bobinées et pour autoriser le bon fonctionnement de l'installation, il est important de contrôler le défilement des nappes de matières de base.

-14-

A cet égard, l'installation de la présente invention comporte des moyens de vérification de présence de matières, moyens connus de l'Homme de l'Art généralement basés sur des systèmes photo-électriques ou des dispositifs à rouleaux flottants ou autres.

Toutefois, il est à remarquer que ces contrôles ne seront pas systématiquement tous rendus automatiques et certains d'entre eux pourront rester sous le contrôle de l'opérateur de la machine. C'est le cas de l'engagement des bobines de pâte 3 ou des bobines de voile de fibres cellulosiques 4 qui ont des vitesses de déroulement relativement faibles.

Par contre, les bobines non tissé 5 et de film étanche polyéthylène 6 seront avantageusement contrôlées automatiquement, de même leur raboutement sera également mécanisé.

A cet égard, la figure 7 propose un dispositif permettant le raboutement des nappes de non-tissé 5 placé au niveau du poste de travail 20.

Plus précisément, la figure 7 montre deux bobines 51 et 52 de non-tissé 5, par exemple, la première bobine 51 étant en utilisation par exemple, et la nappe étant dirigée par les rouleaux 53, 54 vers son utilisation.

Lorsqu'un détecteur est actionné par la fin de cette bobine 51, l'extrémité 55 libre de la bobine 52 est alors raboutée sur la nappe de non-tissé qui se déroule de la bobine 51 comme le montrent les pointillés de la figure 7.

A cet égard, l'extrémité 55 présentera avantageusement une zone adhésive 56 qui sera appliquée par tout moyen mécanique connu selon la flèche 57 pour la solidariser avec la bande en défilement. Ensuite, un dispositif de coupe 58 permettra d'interrompre la continuité de la nappe 5 de la bobine 51. Alors, cette dernière sera remplacée par la nappe de non-tissé 5 provenant de la bobine 52.

Il est à remarquer que ce système peut être adapté également aux bobines de film polyéthylène 6 au niveau du poste 17.

Pendant ces phases de raboutement, il est à remarquer qu'il y aura une surépaisseur au niveau du produit fini soit sur la couche externe 5, soit sur la couche externe 6.

Pour pallier à cet inconvénient et pour éjecter les produits non conformes, fabriqués pendant cette phase, qui présenteront alors une surépaisseur dans les couches externes, l'installation de la présente invention comporte des moyens pour contrôler le raboutement des nappes de matières de base lors des changements de bobines.

Pour ce, ces moyens réagiront sur le système de commande général de l'installation et on mémorisera, comme décrit précédemment, les produits fabriqués non conformes afin de les repérer et de les éjecter après la coupe finale du poste 26.

Par ailleurs, dans certains cas de réalisation, des élastiques méplats 7 sont disposés puis collés sur la couche externe 6 étanche dans le sens longitudinal de la bande en défilement 40. Afin de repérer les produits non conformes dans lesquels l'élastique n'est pas collé sur la couche étanche 6, la présente installation 1 comporte des moyens pour contrôler le dévidement de la bande élastique et pour détecter son retournement continu en mèche.

Lorsqu'il y a une détection de défaut, l'information est envoyée au système de commande de l'installation et on mémorise l'endroit de la bande en défilement 40 où s'est produit le retournement pour évacuer les produits non conformes après leur coupe, et d'autre part pour que l'attention de l'opérateur soit attirée afin de corriger le défaut.

Par ailleurs, dans l'installation de fabrication de la présente invention, pour contrôler la qualité des produits obtenus, il est intéressant de vérifier les formes des produits fabriqués.

A cet égard, la dite installation comporte des moyens pour contrôler, transversalement au sens de déplacement de la bande continue 40, l'épaisseur du dit élément interne 10 afin de détecter et de repérer les produits non conformes présentant une surépaisseur et/ou une absence locale de matière absorbante.

En effet, l'élément absorbant est formé, préalablement dans la machine, de fibres cellulosiques en nappe calibrée. Toutefois, il se peut qu'il y ait arrachement d'une partie de l'élément ou, lors de la découpe, la chute soit accidentellement placée sur l'élément absorbant.

Pour effectuer de tels contrôles, on disposera, comme le montre par exemple la figure 6, au moins trois détecteurs 59, 60 et 61 qui respectivement jaugeront l'épaisseur du premier côté latéral du tampon puis son épaisseur centrale puis l'épaisseur du deuxième côté latéral.

A cet égard, on utilisera des jauges d'épaisseur dont le fonctionnement est basé sur la perturbation d'un faisceau de rayonnement par absorption de la matière. Ces techniques sont généralement connues de l'Homme de l'Art.

De la même manière que précédemment, la position des produits non conformes détectés sera mémorisée pour agir et les évacuer après leur coupe finale.

De plus, on peut également utiliser de mêmes types de détecteurs pour constituer des moyens pour contrôler le décalage transversal de la coupe finale des dits produits par rapport à la position de l'élément absorbant 10 placé selon le pas "p" afin de détecter et de repérer des produits non conformes présentant l'élément absorbant 10 mal centré par rapport aux extrémités longitudinales du dit produit.

En effet, de tels détecteurs pourront jauger d'une part l'épaisseur du tampon 10 puis ensuite la faible épaisseur des deux couches externes 5, 6 solidarisées entre elles au niveau de la presse anatomique 21, il suffira alors de synchroniser la lecture avec le défilement de la bande 40 pour centrer la découpe entre deux éléments absorbants 10 consécutifs.

Un des derniers contrôles de qualité intéressant à réaliser réside dans le contrôle de la symétrie dans le sens longitudinal du produit fabriqué.

A cet égard, la présente installation comporte des moyens pour contrôler le décalage latéral de la bande 40 en défilement par rapport au positionnement des différents postes de travail afin de détecter et de repérer des produits non conformes présentant une dissymétrie dans le sens transversal de la bande. Ces contrôles pourront être effectués par tout type de détecteurs de proximité ou de capteurs optiques permettant de contrôler le centrage de la bande 40 par rapport à un axe de défilement fictif.

Tout défaut de symétrie provoquant alors un appel opérateur pour retoucher le positionnement de la bande et une évacuation des produits fabriqués durant la phase de défaut, les produits étant repérés et mémorisés pour que leur évacuation ait lieu après leur coupe finale.

La figure 8 montre les différents postes de travail placés en fin d'installation c'est-à-dire le poste de coupe finale 26 et le poste d'évacuation des produits coupés conformes 28.

Toutefois, entre ces deux postes de l'installation de fabrication de la présente invention, est intercalé un poste d'éjection des produits non conformes 27 essentiellement constitué par une partie amovible pivotante 62 qui, dans une première position autorise l'évacuation vers le poste 28 des produits conformes et qui dans une seconde position ouvre en quelque sorte une trappe qui dirige les produits non conformes repérés 63 sur la figure 8 vers un bac de réception 29.

Cette trappe est par exemple commandée par un vérin 64 qui est actionné par le système de contrôle général de l'installation en fonction de la détection en amont de cette trappe des produits non conformes.

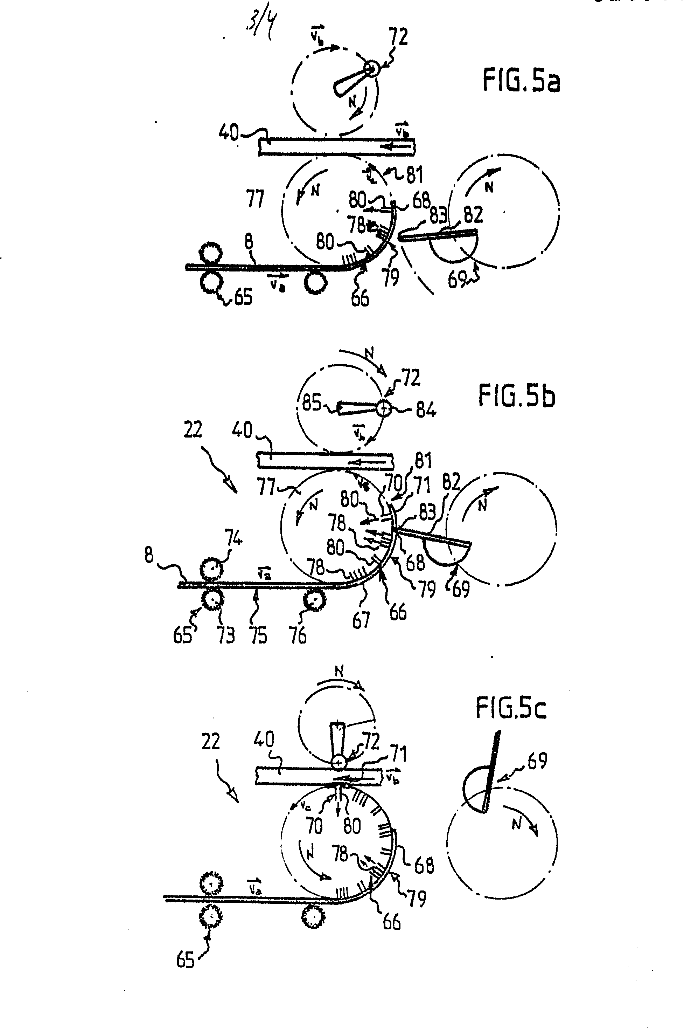

Par ailleurs, selon une autre caractéristique importante de la présente invention, l'installation comporte des moyens de découpe, de positionnement et de pose des adhésifs, synchronisés avec le défilement de la bande en continu et commandés selon le dit pas "p", conçus pour avoir une efficacité maximum et de ce fait pour éviter de donner naissance à des produits non conformes. Ces dits moyens repérés 22 à la figure 1 sont montrés plus en détail aux figures 5a à 5c.

Le ruban adhésif 8 se présente préalablement sous la forme d'une bande adhésive continue disposée par exemple sur un dévidoir non représenté sur la figure. Ce ruban adhésif 8 est ensuite dirigé vers le poste de pose des adhésifs 22 constitué essentiellement par:

a) des moyens d'avance de la bande adhésive 8 délivrant la bande à une vitesse linéaire $v_A$ qui est fonction de la vitesse $v_B$ de défilement de la bande composite 40 et de la longueur "l" du dit adhésif à appliquer,

b) des moyens rotatifs 66 de maintien de la partie 67 de la bande 8 ainsi délivrée dont la vitesse tangentielle $v_C$ est supérieure à la vitesse linéaire $v_A$ d'avance de la bande 8 pour qu'il y ait un léger glissement entre la bande adhésive 8 et les moyens rotatifs 66 de maintien afin de favoriser ce dernier, les dits moyens de maintien 66 et d'avance 65 étant prévus tels qu'ils puissent coopérer entre eux pour préparer l'extrémité 68 de la bande avant la découpe,

c) des moyens de coupe dynamiques 69 de l'extrémité 68 de la bande adhésive 8, ainsi maintenue et préparée, dont l'action est

**0267861**

périodique, selon le pas "p", et asservie à la vitesse tangentielle $v_c$ des moyens rotatifs 66 de maintien, aptes à réaliser une coupe franche de l'extrémité 68 selon la longueur "l" souhaitée,

d) des moyens de transfert dynamiques 70 de la longueur "l" d'adhésif ainsi coupée vers la bande composite 40 en défilement continu, aptes à positionner le dit adhésif découpé 71 sur la dite bande 40 selon le pas "p",

e) des moyens 72 pour appliquer et solidariser le dit adhésif découpé 71 sur la dite bande 40 coopérant de manière synchrone avec les dits moyens de transfert 70.

Selon un mode de réalisation de la présente invention, les moyens d'avance 65 de la bande adhésive 8 sont constitués par au moins deux cylindres 73, 74, dont les génératrices sont sensiblement appliquées l'une contre l'autre, et commandés par un organe moteur de façon à ce que la bande adhésive 8, entrainée entre les deux cylindres 73, 74, se déplace à la dite vitesse linéaire $v_A$.

De plus, le cylindre 73 en regard de la face adhésive 75 de la bande 8 est prévu et traité anti-adhésif pour qu'il n'y ait pas d'adhérence sur le cylindre. Par exemple, il présentera une périphérie taillée en "pointes de diamant"; de plus, la surface sera recouverte de téflon. Par contre, le cylindre 74 peut être lisse car il voit l'autre face, non adhésive.

De plus, pour autoriser un meilleur guidage, le dispositif pourra présenter un autre cylindre 76 du même type que le dit cylindre 73.

Par ailleurs, selon le mode de réalisation représenté à la figure 5, les moyens rotatifs 66 de maintien et de préparation de l'extrémité 68 de la bande adhésive 8 à couper, et les moyens de transfert dynamiques 70 de la longueur "l" d'adhésif découpé 71 se présentent sous la forme d'une roue à vide 77 dont l'espace intérieur est soumis à une dépression et dont la périphérie est percée d'une multitude d'orifices.

A cet égard, la périphérie est percée d'une première série de dits premiers orifices 78 qui sont répartis uniformément le long de la périphérie et qui mettent en communication l'espace intérieur et l'extérieur de la roue 77. De plus, ces orifices sont commandés sélectivement sur au moins un premier secteur 79 de la périphérie afin de constituer substantiellement les dits moyens de maintien et de préparation 66 de l'extrémité de la bande.

De plus, la périphérie de la roue 77 est également percée d'un ou plusieurs deuxièmes orifices 80 qui sont répartis d'une manière alternée par rapport à la dite première série d'orifices 78 et qui mettent également en communication l'espace intérieur et l'extérieur de la roue. Ce ou ces dits deuxièmes orifices 80 sont également commandés sélectivement sur au moins un deuxième secteur 81 contigu au dit premier secteur 79 dans le sens de rotation de la roue afin de constituer substantiellement les dits moyens de transfert 70 de la longueur "l" d'adhésif découpé 71.

En ce qui concerne les moyens de coupe dynamiques 69, ils sont dans le cas de la figure 5 constitués par un couteau 82 dont le fil de coupe 83 est au moins tangent en un point avec la périphérie de la roue à vide 77 comme le montre particulièrement la figure 5b.

En outre, le couteau 82 est animé d'un mouvement de rotation asservi à celui de la roue à vide 77 pour former par coopération roue-couteau la dite coupe franche de l'adhésif au point de tangence.

Enfin, les moyens 72 pour appliquer et solidariser le dit adhésif découpé 71 sont avantageusement constitués par une roulette d'appui 84, libre sur son axe, disposée telle qu'elle effectue un mouvement de rotation synchronisé avec la vitesse de rotation N de la roue à vide 77 autour d'un axe 85 parallèle à celui de la roue à vide 77.

De plus, lorsque la roulette 84 est en vis-à-vis avec la roue à vide 77, l'écart entre elles est tel que la bande composite 40 continue puisse défiler entre elles et soit légèrement comprimée pour solidariser l'adhésif 71 sur la bande 40 selon le pas "p", comme le montre notamment la figure 5c.

Il est à remarquer que deux fonctionnements du dispositif de coupe des adhésifs sont possibles. D'une part, on peut prévoir la coupe toujours au même endroit de la roue à vide 77, d'autre part, on peut prévoir un point d'impact différent à chaque tour de roue à vide 77.

Dans le cas où on prévoit une coupe d'adhésif à chaque tour des moyens rotatifs, c'est-à-dire de la roue 77, il y aura lieu d'asservir l'action périodique des moyens de coupe 69 à la vitesse de rotation N des moyens rotatifs 66 de maintien. Plus précisément, dans ce cas, les deux vitesses de rotation seront par exemple identiques, de même pour la vitesse tangentielle $v_c$ de la roue 77 et celle du fil de coupe 83. Dans ce cas, la matière de la roue 77 sera avantageusement choisie parmi les aciers spéciaux de très grande dureté.

Par contre, si l'on désire utiliser des matériaux plus classiques, pour éviter le marquage de la roue 77, on effectuera des coupes d'adhésif à différents endroits de la roue, et il y aura lieu d'asservir l'action périodique des moyens de coupe pour qu'il y ait coupe d'adhésif par exemple à chaque 7/8 ème de tour des moyens rotatifs 77.

Le fonctionnement du dispositif de pose et d'adhésif 22 est notamment illustré aux figures 5a à 5c.

La figure 5a montre la préparation de l'extrémité 68 de la bande adhésive qui est maintenue plaquée contre la roue 77 par l'aspiration au-travers des orifices 78 et 80.

Lorsque la longueur "1" de l'adhésif 71 à découper est correcte, la bande étant toujours maintenue appliquée contre la roue 77 par l'aspiration au-travers des orifices 78 et 80, la rotation synchronisée de son couteau 82 fait que son fil de coupe 83 se présente juste tangent à la roue à vide 77 pour effectuer une coupe franche.

Comme le montre la figure 5c, le morceau d'adhésif 71 est ensuite transféré vers la bande en défilement 40 d'une manière synchronisée par rapport au pas "p" par l'intermédiaire de l'orifice 70 qui alors est commandé sélectivement jusqu'à la dépose de l'adhésif sur la bande 40. Par contre, l'extrémité 68 de la bande adhésive se reprépare pour une coupe suivante, cette dernière est maintenue par d'autres orifices 78 qui sont alors commandés dans le secteur 79, les premiers orifices de maintien étant alors déconnectés de l'aspiration.

Avantageusement, la vitesse de rotation des moyens 66 de maintien de la bande adhésive, celle des moyens de transfert 70, celle des moyens de coupe 69 et celle des moyens 72 pour appliquer et solidariser le dit adhésif 71 sur la bande 40 seront avantageusement identiques et telles qu'un tour de rotation de ces différents organes corresponde au développé du pas "p".

Naturellement, d'autres systèmes de coupe pourraient être

0267861

-21-

envisagés mais toutefois il est à remarquer que ce dispositif de coupe est avantageux car il permet de minimiser les erreurs de pose d'adhésif et par suite de réduire le nombre de produits non conformes.

Par ailleurs, d'autres modes de réalisation de la présente invention, à la portée de l'Homme de l'Art, pourraient être envisagés pour constituer les différents moyens spécifiques de la présente invention et ce sans sortir du cadre de la présente invention.

REVENDICATIONS

1. Installation de fabrication (1) de produits plats en forme (2), tels que notamment des changes jetables pour bébés ou pour incontinents, constituée de différents postes de travail successifs (11 à 29) en ligne et comprenant au moins :

- des moyens pour former, à partir de diverses matières de base (3 à 8) en nappes bobinées, une bande continue composite (40) présentant au moins deux couches externes (5 et 6), à savoir une couche (6) continue étanche au liquide et une couche (5) continue présentant un toucher confortable vis-à-vis de la peau, et présentant un élément (10) en forme, perméable et absorbant, placé entre les deux dites couches externes (5 et 6), selon un pas prédéterminé "p",

- des moyens pour solidariser au moins partiellement les deux couches externes (5 et 6) et le dit élément absorbant (10) entre eux, caractérisée par le fait qu'elle comporte :

- des moyens pour contrôler le défilement des nappes de matières de base (3 à 8),

- des moyens pour contrôler, de manière synchrone, la bande composite (40) ainsi formée en défilement, disposés aux différents postes de travail de l'installation (11 à 29), selon des paramètres de formes et de qualité du produit à obtenir (2),

- des moyens pour repérer, selon le pas "p", les produits obtenus (2) non conformes aux dits paramètres,

- des moyens pour découper la bande continue (40) ainsi formée, selon le pas "p", pour produire les produits plats en forme (2),

- des moyens pour éjecter, après leur découpage, les produits obtenus (2) repérés non conformes.

2. Installation de fabrication, selon la revendication 1, des longueurs prédéterminées de ruban adhésif (8) étant appliquées sur au moins une des deux couches externes de la bande (5 ou 6) de la bande continue (40), au niveau de chacun de ses côtés latéraux, pendant son défilement et selon un pas pré-établi "p", caractérisée par le fait qu'elle comporte des moyens (45) pour contrôler la présence des adhésifs (8) après leur pose ainsi que leur positionnement, de manière synchrone par rapport au défilement de la bande (40), afin de détecter ou repérer les produits obtenus (2) non

-23-

conformes sans adhésif et/ou avec adhésif mal positionné.

3. Installation de fabrication selon la revendication 1, caractérisée par le fait qu'elle comporte des moyens pour contrôler la vitesse de défilement de la bande continue (40) afin de détecter et repérer les produits obtenus (2) non conformes formés pendant les régimes transitoires du fonctionnement de la dite installation (1).

4. Installation de fabrication selon la revendication 1, caractérisée par le fait qu'elle comporte des moyens pour contrôler le raboutement des nappes de matières de base (5 et/ou 6) lors des changements de bobines afin de détecter et repérer les produits obtenus (2) non conformes présentant une surépaisseur dans les couches externes (5, 6).

5. Installation de fabrication selon la revendication 1, des élastiques méplats (7) étant disposés puis collés sur la couche externe étanche (6) dans le sens longitudinal de la bande (40) en défilement, caractérisée par le fait qu'elle comporte des moyens pour contrôler le dévidement de la bande élastique (7) et pour détecter son retournement continu en mèche afin de repérer les produits obtenus (2) non conformes dans lesquels l'élastique (7) n'est pas collé sur la couche étanche (6).

6. Installation de fabrication selon la revendication 1, l'élément absorbant (10) étant formé de fibres cellulosiques en nappe calibrée, caractérisée par le fait qu'elle comporte des moyens (59 à 61) pour contrôler, transversalement au sens de déplacement de la bande continue (40), l'épaisseur du dit élément interne (10) afin de détecter et repérer les produits obtenus (2) non conformes présentant une surépaisseur et/ou une absence locale de matière absorbante.

7. Installation de fabrication selon la revendication 1, caractérisée par le fait qu'elle comporte des moyens pour contrôler le décalage latéral de la bande (40) en défilement par rapport au positionnement des différents postes de travail (11 à 29) afin de détecter et de repérer des produits obtenus (2) non conformes présentant une dissymétrie dans le sens transversal du dit produit (2).

8. Installation de fabrication selon la revendication 1, la longueur de l'élément absorbant (10) étant inférieure au pas "p", caractérisée par le fait qu'elle comporte des moyens pour contrôler le décalage transversal de la coupe finale des dits produits (2) par rapport à la position de l'élément absorbant (10) placé selon le pas "p", afin de détecter et de repérer des produits obtenus (2) non conformes présentant l'élément absorbant (10) mal centré par rapport aux extrémités longitudinales du dit produit (2).

9. Installation de fabrication, selon la revendication 1, des longueurs prédéterminées de ruban adhésif (8) étant appliquées sur au moins une des deux couches externes de la bande (5 ou 6) de la bande continue (40), au niveau de chacun de ses côtés latéraux, pendant son défilement et selon un pas pré-établi "p", le ruban adhésif (8) se présentant préalablement sous la forme d'une bande adhésive continue disposée par exemple sur un dévidoir, caractérisée par le fait qu'elle comporte des moyens de découpe, de positionnement, et de pose des adhésifs, synchronisés avec le défilement de la bande continue (40) et commandés selon le dit pas "p", constitués par :

a) des moyens d'avance (65) de la bande adhésive (8) délivrant la bande à une vitesse linéaire $v_A$ qui est fonction de la vitesse $v_B$ de défilement de la bande composite (40) et de la longueur "l" du dit adhésif à appliquer,

b) des moyens rotatifs (66) de maintien de la partie (67) de la bande (8) ainsi délivrée dont la vitesse tangentielle $v_C$ est supérieure à la vitesse $v_A$ d'avance de la bande adhésive (8) pour qu'il y ait un léger glissement entre la bande adhésive (8) délivrée et les moyens rotatifs (66) de maintien pour favoriser ce dernier,

- les dits moyens de maintien (66) et d'avance (65) coopérant entre eux pour préparer l'extrémité (68) de la bande adhésive (8) avant la coupe,

c) des moyens de coupe dynamiques (69) de l'extrémité (68) de la bande adhésive (8) ainsi maintenue et préparée, dont l'action est périodique selon le pas "p" et asservie avec la vitesse tangentielle $v_C$ des moyens rotatifs (66) de maintien, aptes à réaliser une coupe franche de l'extrémité (68) selon la longueur "l" souhaitée, pour former successivement les dites longueurs prédéterminées (71) de ruban adhésif.

10. Installation de fabrication selon la revendication 9, caractérisée par le fait que les moyens de découpe, de positionnement et de pose des adhésifs comportent en outre :

a) des moyens de transfert dynamiques (70) de la longueur

"l" d'adhésif (71) ainsi coupée vers la bande composite (40) en défilement continu, aptes à positionner le dit adhésif (71) sur la dite bande (40) selon le pas "p",

b) des moyens (72) pour appliquer et solidariser le dit adhésif (71) sur la dite bande (40), synchronisés avec le défilement de la bande (40) et coopérant de manière synchrone avec les dits moyens de transfert (70).

11. Installation de fabrication selon la revendication 10, caractérisée par le fait que les moyens rotatifs (66) de maintien et de préparation de l'extrémité (68) de la bande adhésive (8) à couper et les moyens de transfert dynamiques (70) de la longueur "l" d'adhésif découpé (71) se présentent sous la forme d'une même roue à vide (77) dont l'espace intérieur est soumis à une dépression, et dont la périphérie est percée d'une première série de dits premiers orifices (78), répartis uniformément et mettant en communication l'espace intérieur et l'extérieur de la roue (77) par une commande sélective sur au moins un premier secteur (79) de la périphérie de la roue (77) afin de constituer substantiellement les dits moyens de maintien de préparation (66) de l'extrémité de la bande (68), et percée d'un ou plusieurs dits deuxièmes orifices (80) répartis d'une manière alternée par rapport à la dite première série d'orifices (78), mettant en communication l'espace intérieur et l'extérieur de la roue (77) par une commande sélective sur au moins un deuxième secteur (81) contigu au dit premier secteur (79), afin de constituer substantiellement les dits moyens de transfert (70) de la longueur "l" d'adhésif découpé (71).

12. Installation de fabrication selon la revendication 9, caractérisée par le fait que les moyens d'avance (66) de la bande adhésive (8) constitués par au moins deux cylindres (73, 74) dont les génératrices sont sensiblement appliquées l'une contre l'autre, commandés par un organe moteur de façon à ce que l'adhésif (8) entrainé entre les deux cylindres (73, 74) se déplace avec une vitesse linéaire $v_A$, le cylindre (73), en regard de la face adhésive (75) du dit ruban (8), étant prévu et traité anti-adhésif pour qu'il n'y ait pas d'adhérence sur le cylindre (73).

13. Installation de fabrication selon la revendication 11, caractérisée par le fait que les moyens de coupe dynamiques (69) sont constitués par un couteau (82) dont le fil de coupe (83) est au moins tangent en un point avec la périphérie de la roue à vide (77) pour former la dite coupe franche de l'adhésif en ce point et, animé d'un mouvement de rotation asservi à celle de la roue à vide (77).

# FIG.1

20

3

2  28   27 2  40 25   23 22     5   16  15   14   32      2

29  26   24   8  40 21 17 6 18 7 19   13  4   12      11

1/4

# FIG.2

2

8

5

7

6

3

4

9

10

# FIG.3

2

8

5

7

6

0267861

FIG.4a
FIG.4b
FIG.4c
FIG.4d
FIG.4e

6267861

2/4

0267861

FIG.5a

FIG.5b

FIG.5c

0267861

4/4

FIG.6

FIG.8

FIG.7